# EUROPÄISCHE PATENTSCHRIFT

(11) **EP 1 296 809 B1**
(45) Veröffentlichungstag und Bekanntmachung des Hinweises auf die Patenterteilung: **13.10.2004**
(21) Anmeldenummer: 01949465.7
(22) Anmeldetag: 04.07.2001
(51) Int. Cl.: B29C 43/36, B29C 33/38, B22C 9/00, A61F 2/30

(54) **VERFAHREN ZUR NATURGETREUEN HERSTELLUNG MEDIZINISCHER IMPLANTATE UND EPITHESEN UND DANACH HERGESTELLTE IMPLANTATE UND EPITHESEN**
METHOD FOR THE PRODUCTION OF FAITHFULLY-REPRODUCED MEDICAL IMPLANTS AND EPIPROSTHESES AND SAID IMPLANTS AND EPIPROSTHESES
PROCEDE DE FABRICATION D'IMPLANTS MEDICAUX ET D'EPITHESES PAR REPRODUCTION FIDELE, IMPLANTS ET EPITHESES OBTENUS SELON LEDIT PROCEDE

(30) Priorität: 04.07.2000 DE 10033313
(43) Veröffentlichungstag der Anmeldung: 02.04.2003
(73) Patentinhaber: 3di GmbH, 07745 Jena (DE)
(72) Erfinder: LITSCHKO, Peter, 06618 Naumburg (DE); NAGEL, Sebastian, 07743 Jena (DE); KÖRBS, Thomas, 07629 Hermsdorf (DE); SCHIED, Ralf, 06618 Naumburg (DE)
(74) Vertreter: Pfeiffer, Rolf-Gerd
(86) Internationale Anmeldenummer: PCT/EP2001/007651
(87) Internationale Veröffentlichungsnummer: WO 2002/002030

(56) Entgegenhaltungen:
- WO-A-93/16865
- DE-A- 4 102 256
- DE-A- 4 102 258
- DE-A- 4 409 836
- DE-A- 19 614 949
- DE-C- 19 518 994
- US-A- 4 097 935
- US-A- 4 976 737
- US-A- 5 133 771
- US-A- 5 370 692
- US-A- 5 824 078
- US-A- 6 022 509

## Beschreibung

Die Erfindung betrifft ein Verfahren zur naturgetreuen Herstellung medizinischer Implantate und eine danach hergestellte Negativform gemäß der Gattung der Patentansprüche 1 und 8. Wenn in der nachfolgenden Beschreibung und den Ansprüchen von Implantaten die Rede ist, sind Epithesen grundsätzlich eingeschlossen.

In der Medizin ist die Herstellung von Implantaten oder Epithesen seit langem bekannt, wobei diese während oder vor einer Operation ihre endgültige Form erhalten. Aus den US-A 4,097,935 und US-A 4,976,737 ist es bekannt, begrenzt formbare bzw. bearbeitbare Metallnetze, Metallplatten o.a. manuell formbare Materialien zur Implantation zu verwenden, d.h. das Implantat wird während der Operation durch den behandelnden Operateur geformt, bearbeitet und angepaßt. Die Ergebnisse sind jedoch sowohl in funktioneller, als auch in ästhetischer Hinsicht häufig nicht optimal, da die Implantate bzw. Epithesen rein manuell, unter Einsatz einfachster Werkzeuge, in Handarbeit gefertigt werden müssen.
Darüber hinaus ist es bekannt, aus den Daten bildgebender Untersuchungsverfahren eines Patienten ein 3D-Modell zu generieren und aus diesem, unter Verwendung konstruktiver Methoden, das Positiv-Implantat virtuell am Rechner zu entwerfen.
US-A 5,824,078 beschreibt eine Methode, mit Hilfe derer vermittels Pressen eine Anpassung und Oberflächenausformung eines zusammengesetzten artgleichen Implantats, insbesondere einer Hüftgelenkpfanne erfolgt.
Vorliegender Erfindung gattungsmäßig nahekommende Schriften sind DE-A 41 02 256 und DE-A 41 02 258. Insbesondere letztere beschreibt ein Verfahren zur Herstellung einer Negativform beliebig geformter Teile, bei dem vermittels eines Laserstrahls, dessen Auslenkung anhand von computertomografisch erfasster Patientendaten erfolgt, eine Aushärtung von Konturen zwecks Bildung der Form vorgenommen wird.

Der Erfindung liegt die Aufgabe zu Grunde, ausgehend von vorhandenen Daten eines Patienten individuelle medizinische Implantate aus geeigneten Materialien, individuell angepasste Implantate in relativ einfacher Weise genau herzustellen, die gehobenen funktionellen und ästhetischen Ansprüchen genügen.

Erfindungsgemäß wird diese Aufgabe durch das Verfahren nach Anspruch 1 und durch die Negativform nach Anspruch 8 gelöst. Vorteilhafte Weiterbildungen der Erfindung sind Gegenstand der betreffenden abhängigen Ansprüche.

Das virtuelle dreidimensionale Modell des medizinischen Implantats wird in bekannter Weise aus vorliegenden Daten des Patienten oder durch irgendeine andere Form der Konstruktion gebildet. Es bildet eine Vorrichtung, welche das zukünftige Implantatvolumen umschliesst (Hohlform). Die individuelle Hohlform wird erzeugt durch die Auswertung der zur Verfügung gestellten Patientendaten bzw. der Daten des virtuellen Implantats erzeugt. Aus diesen Daten werden Daten für die computergesteuerte Fertigung (z.B. CNC-Fräsen) abgeleitet. Die Ausgangsmaterialien zur Herstellung der Hohlform richten sich nach dem späteren Material für das Implantat, das bevorzugt aus Metall- bzw. Keramikhohlformen für das Design von Implantaten aus körperfremden Materialien und biopassivem Glas für körpereigene Materialien besteht.
In die Hohlform, die zur einfacheren Entformung sowohl trennbar, als auch aus einem Stück und aus unterschiedlichen Materialien bestehen kann, wird anschließend das eigentliche Implantatsmaterial eingebracht. Dieses kann sowohl körperfremdes Material (Metalle, Keramiken, Kunststoffe usw.) in pulverförmigen oder flüssigem Zustand sein, als auch körpereigenes Material (z.B. körpereigene Zellen in einer Nährstofflösung).
Im Fall des körperfremden Materials wird nach abschließenden technischen Verfahrensschritten (z.B. Sintern, Pressen, Temperaturbehandlung usw.) das fertige Implantat der Form entnommen und ggf. nachbearbeitet. Besondere Bedeutung hat die Anwendung technologischer Prozesse, wie z.B. das Aufschäumen der Materialien während der Formgebung, um dem individuellen Implantat näturähnliche Eigenschaften (knochenähnliche Strukturen) zu geben. Handelt es sich um körpereigenes Material, so wächst das individuelle Implantat in der Negativform unter Berücksichtigung biologischer und physikalischer Parameter zu einem Implantat aus körpereigenem Material heran. Beispielsweise werden dazu dem Patienten zunächst körpereigene Gewebezellen (Knorpel, Knochen usw.) entnommen und diese zusammen mit entsprechender Nährlösung und evtl. weiterer Materialien (z.B. poröse Trägerkörper aus vom Körper resorbierbaren und ersetzbaren Materialien) in die entwickelte und hergestellte individuelle, als Hohlform gestaltete Negativform eingebracht, die aus einem Material gefertigt ist, das mit dem Implantatsmaterial verträglich ist. Aus den Komponenten entwickelt sich so das Implantat. Auf diese Weise kann das medizinische Individualimplaatat unter ästhetischen und funktionellen Aspekten in beliebiger Form, Größe und Kompliziertheit aus vielfältigen, unterschiedlichen Werk- bzw. Naturstoffen gefertigt werden. Ein weiterer Vorteil besteht in der individuellen objektiven Formgebung durch Berücksichtigung der unterschiedlichsten Patientendaten bei der Gestaltung der Hohlform für das Implantat.

Die Erfindung wird nachstehend an Hand der schematischen Zeichnung näher erläutert. Es zeigen:
Fig. 1 die Erstellung eines virtuellen Modells des Implantats,
Fig. 2 die Herstellung einer Negativform zum Implantat,
Fig. 3 die Füllung der Negativform mit Implantatsmaterial und
Fig. 4 ein fertiges Implantat.

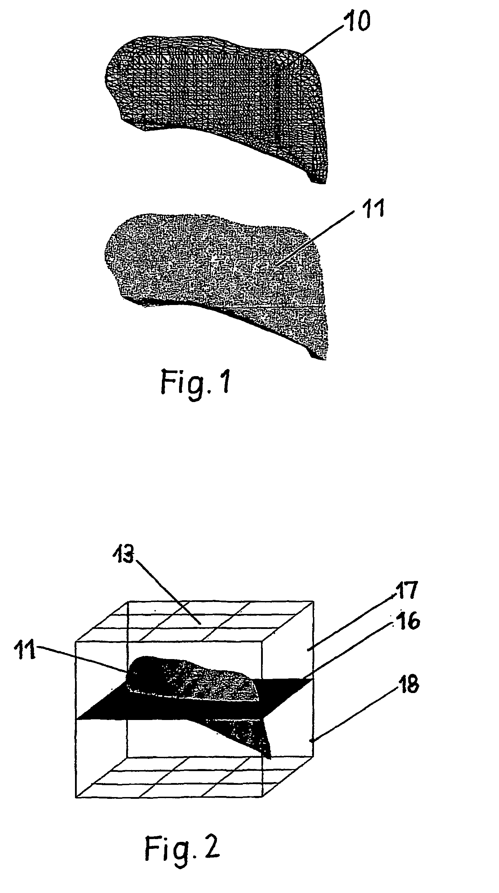

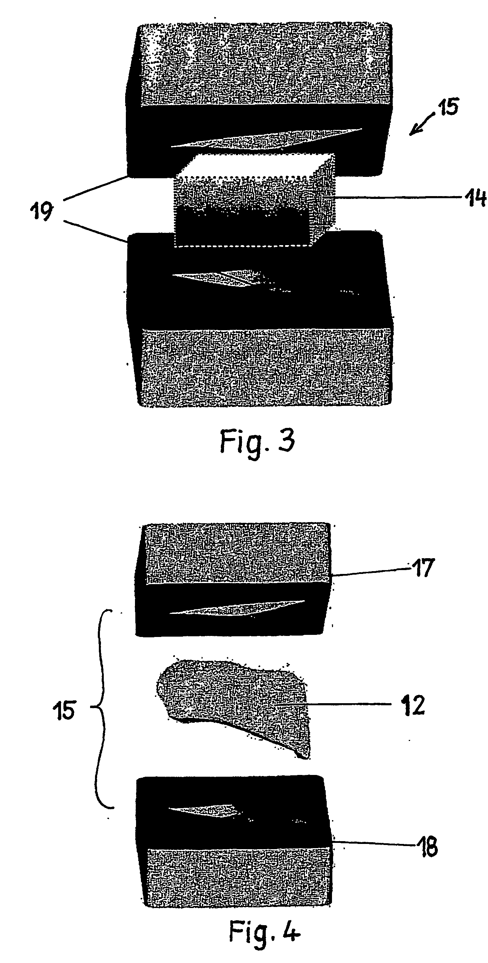

Ausgehend von den Daten bildgebender medizinischer Verfahren, bspw. der Computertomografie, wird in an sich bekannter Weise mittels Computer für einen Patienten individuell ein passendes Implantatmodell konstruiert, das als Flächenmodell 10 entstehen kann und in ein Volumenmodell 11 umgewandelt wird (Fig. 1). Zur Herstellung einer realen Negativform 15 eines herzustellenden Implantates 12 wird per Computer ein das Implantatmodell 11 umgebendes Volumen 13 ermittelt (Fig.2). Durch Bilden der Differenz zwischen dem umgebenden Volumen 13 und dem Volumenmodell 11 wird die Negativform grundsätzlich beschrieben und konstruiert. Um die Einbringung eines Implantatmaterials 14 (Fig. 3) in die Negativform zu gewährleisten, wird die Konstruktion der Fig. 2 durch mindestens eine Schnittebene 16 in Formteile 17, 18 zerlegt. Die so gewonnen Konstruktionsdaten für die Formteile 17, 18 werden in einen für den Herstellungsprozess geeignetes Datenformat umgewandelt. Bspw. werden sie in Steuerungsdaten für eine nicht dargestellte computergesteuerte Fräsmaschine umgesetzt.
Nach der Fertigung der aus den Formteilen 17, 18 bestehenden Negativform 15 wird in deren Hohlraum 19 das erforderliche Implantatmaterial 14, z. B. ein (Kunststoff-) Pulver oder Pulver-Binder-Gemisch, eingebracht und gepresst.

In den Hohlraum 19 der Negativform 15 können auch körpereigene Zellen, Nährstofflösung und Trägermaterial eingebracht werden, wodurch in der Form ein induziertes Wachstum des Implantatmaterials zustande käme.
Der Ausgangswerkstoff für die Fertigung der Negativform 15 wird für das Anwendungsgebiet entsprechend ausgewählt und ist auf das Implantatmaterial 14 abgestimmt. Bspw. werden Hohlformen aus Metall oder Keramik für die Herstellung von Implantaten aus körperfremden Materialien und Hohlformen aus biopassivem Glas für Implantate aus körpereigenen Materialien verwendet.
Die aus einem Stück oder aus Formteilen bestehende Negativform kann auch Bohrungen oder Zugänge aufweisen, um flüssige Implantatmaterialien oder Nährstofflösungen zuführen zu können, wobei letztere das Wachstum eines im Hohlraum 19 befindlichen Implantats anregen bzw. ermöglichen und ggf. kontinuierlich zugeführt werden müssen.
Nach Abschluss der Formgebung werden die Formteile 17, 18 auseinandergeklappt und das Implantat 12 aus der Negativform 15 entnommen. Das Implantat 12 kann erforderlichenfalls noch einer Nachbehandlung, z. B. Sintern, unterzogen werden.

### Bezugszeichenliste

- 10: Flächenmodell
- 11: Volumenmodell
- 12: Implantat
- 13: Volumen
- 14: Implantatmaterial
- 15: Negativform
- 16: Schnittebene
- 17, 18: Formteile
- 19: Hohlraum

## Patentansprüche

1. Verfahren zur naturgetreuen Herstellung medizinischer Implantate bei dem aus vorliegenden Daten eines jeweiligen Patienten ein virtuelles Modell erstellt wird, **dadurch gekennzeichnet, dass**
• um dieses virtuelle Modell ein umgebendes Volumen ermittelt wird,
• durch Differenzbildung von umgebenden Volumen und virtuellem Modell eine Negativform beschrieben wird, welche durch eine geeignete Schnittebene getrennt wird,
• die Daten der beschriebenen Negativform in ein für den Herstellungsprozess geeignetes Datenformat umgewandelt werden und
• im Anschluss daran die Herstellung einer realen Negativform erfolgt, in die das Implantatsmaterial eingebracht wird.

2. Verfahren gemäß Anspruch 1, **dadurch gekennzeichnet, dass** das Implantatsmaterial körperfremdes Material ist.

3. Verfahren gemäß Anspruch 1, **dadurch gekennzeichnet, dass** das Implantatsmaterial körpereigenes Material ist.

4. Verfahren gemäß Anspruch 2, **dadurch gekennzeichnet, dass** das körperfremde Material pulverförmig ist und gepresst wird.

5. Verfahren gemäß Anspruch 2, **dadurch gekennzeichnet, dass** das körperfremde Material sich in einem flüssigen Zustand befindet.

6. Verfahren gemäß Anspruch 2, **dadurch gekennzeichnet, dass** das körperfremde Material während der Formgebung aufgeschäumt wird.

7. Verfahren gemäß Anspruch 3, **dadurch gekennzeichnet, dass** körpereigene Gewebezellen mit entsprechender Nährlösung und ggf. weiteren resorbierbaren Materialien in die Negativform eingebracht werden.

8. Negativform, die gemäß den Ansprüchen 1 - 7 hergestellt wird, **dadurch gekennzeichnet, dass** sie eine Hohlform und aus einem Material gefertigt ist, das mit dem Implantatsmaterial verträglich ist.

9. Negativform gemäß Anspruch 8, **dadurch gekennzeichnet, dass** sie aus Teilen besteht.

10. Negativform gemäß Anspruch 9, **dadurch gekennzeichnet, dass** ihre einzelnen Teile aus unterschiedlichen Materialien bestehen.

## Claims

1. Method for the production of faithfully reproduced medical implants, for which a virtual model is prepared from the existing data of a particular patient, **characterized in that**
- a volume surrounding said virtual model is determined
- a negative mold is defined by forming the difference between the surrounding volume and the virtual model being separated by a suitable sectional plane
- the data of the defined negative model are converted into a data format suitable for the production process,
- subsequently, producing an actual negative mold into which the implant material is introduced.

2. Method as claimed in claim 1, **characterized in that** the implant material is an exogenous material.

3. Method as claimed in claim 1, **characterized in that** the implant material is an endogenous material.

4. Method as claimed in claim 2, **characterized in that** the exogenous material is powdery and will be pressed.

5. Method as claimed in claim 2, **characterized in that** the exogenous material is in a liquid state.

6. Method as claimed in claim 2, **characterized in that** the exogenous material is foamed during the molding process.

7. Method as claimed in claim 3, **characterized in that** endogenous tissue cells are introduced into the negative mold together with an appropriate nutrient solution and, if required, further absorbable materials.

8. A negative mold, which is produced in accordance to the claims 1-7, **characterized in that** it is a hollow mold and produced from a material, which is compatible with the implant material.

9. A negative mold as claimed in claim 8, **characterized in that** it consists of parts.

10. A negative mold as claimed in claim 9, **characterized in that** the individual parts of the mold consist of different materials.

## Revendications

1. Procédé permettant la fabrication d'implants médicaux reproduisant fidèlement les conditions naturelles, à partir d'un modèle virtuel basé sur les données personnalisées prélevées d'un patient donné, est **caractérisé en ce que**
- est d'abord déterminé le volume entourant le modèle virtuel, la différence entre le volume entourant et le modèle virtuel décrit un moule en négatif divisé en deux par un plan de coupe approprié,
- les données du moule en négatif décrit sont transformées en un format de données approprié au processus de formage
- le moule en négatif réel dans lequel le matériau d'implant sera injecté, est ensuite façonné.

2. Le procédé suivant la revendication 1 est **caractérisé en ce que** le matériau de l'implant est un matériau ne provenant pas du corps du patient.

3. Le procédé suivant la revendication 1 est **caractérisé en ce que** le matériau de l'implant est un matériau provenant du corps du patient.

4. Le procédé suivant la revendication 2 est **caractérisé en ce que** le matériau non provenant du corps humain est utilisé à l'état pulvérulent pour être pressé.

5. Le procédé suivant la revendication 2 est **caractérisé en ce que** le matériau non provenant du corps humain est utilisé à l'état liquide.

6. Le procédé suivant la revendication 2 est **caractérisé en ce que** lors du formage de l'implant le matériau non provenant du corps humain subit un traitement de moussage.

7. Le procédé suivant la revendication 3 est **caractérisé en ce que** les cellules du tissu provenant du corps humain et une solution nutritive appropriée et que, le cas échéant, d'autres matériaux résorbables sont également introduits dans le moule en négatif.

8. Le moule en négatif façonné suivant les revendications 1 à 7, est **caractérisé en ce qu'**il s'agit d'un moule en creux fabriqué dans un matériau compatible avec les matériaux de l'implant.

9. Le moule en négatif suivant la revendication 8 est **caractérisé en ce qu'**il se compose de plusieurs éléments.

10. Le moule en négatif suivant la revendication 9 est **caractérisé en ce que** ses différents éléments sont fabriqués dans des matériaux différents.
